# EUROPEAN PATENT APPLICATION

(11) **EP 2 016 945 A1**
(43) Date of publication of application: **21.01.2009**
(21) Application number: 06843071.9
(22) Date of filing: 22.12.2006
(51) Int. Cl.: A61K 31/5377, A61K 31/56, A61K 45/00, A61P 11/02, A61P 11/06, A61P 27/16, A61P 37/00, A61P 37/08, C07D 277/20, C07D 277/42

(54) **PHARMACEUTICAL CONTAINING THIAZOLE DERIVATIVE AS ACTIVE INGREDIENT**

(30) Priority: 11.05.2006 JP 2006133125
(71) Applicant: Dainippon Sumitomo Pharma Co., Ltd., Osaka 541-8524 (JP)
(72) Inventor: SUGASAWA, Toshinari, Suita-shi, Osaka 564-0053 (JP); NAKAJIMA, Takashi, Osaka-shi, Osaka 554-0022 (JP)
(74) Representative: Vossius & Partner
(86) International application number: PCT/JP2006/325614
(87) International publication number: WO 2007/132546

(57) **Abstract**

A prophylactic and/or therapeutic agent for sinusitis, nasal obstruction· nasal mucosa congestion containing N-{2-[2-[(3-fluorophenyl)imino]-4-(4-morpholin-4-ylphenyl)-1,3-thiazol-3(2H)-yl]ethyl}-N'-methylurea represented by the formula (1) or its pharmaceutically acceptable salt as an active ingredient.

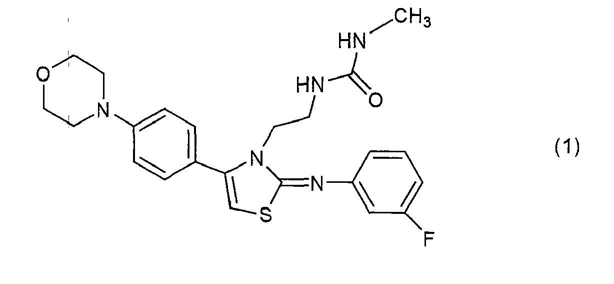

## Description

### TECHNICAL FIELD

The present invention relates to a medicament containing N-{2-[2-[(3-fluorophenyl)imino]-4-(4-morpholin-4-ylphenyl)-1,3-thiazol-3(2H)-yl]ethyl}-N'-methylurea, which is represented by the following formula (1): 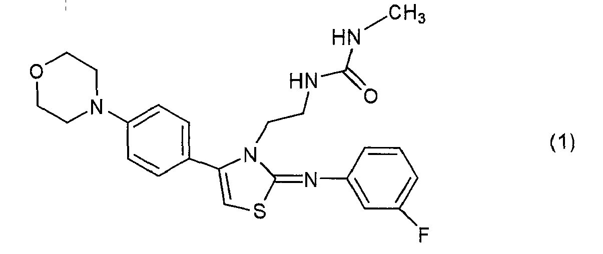 or its pharmaceutically acceptable salt, as an active ingredient.

In more detail, the present invention relates to a medicament containing as an active ingredient, the above compound (1) or its pharmaceutically acceptable salt useful as a prophylactic and/or therapeutic agent for sinusitis, nasal obstruction nasal mucosa congestion. Furthermore, the present invention relates to a medicament containing as an active ingredient, the above compound (1) or its pharmaceutically acceptable salt useful as a prophylactic and/or therapeutic agent for asthma.

### BACKGROUND OF ART

In Patent document 1, there is described a compound represented by the following formula (2), but it is not described therein that the compound is effective for nasal obstruction while no agent is effective for said disease in case of oral administration. And it is neither described therein that the compound is effective for sinusitis.

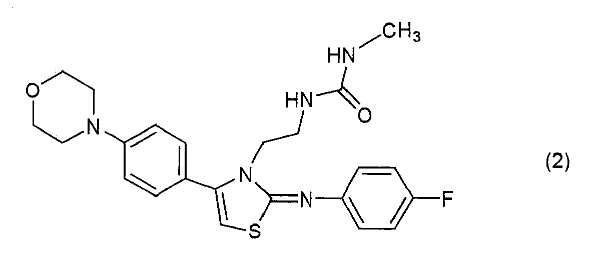

Immediate and delayed reactions on nasal obstruction symptom are known as well as on asthma, but nasal obstruction belongs to nasal mucosa vessel reaction different from asthma, and it is considered that nasal obstruction occurs due to edema·oncoma of nasal sinus mucosa. Different from reaction of transient immediate nasal obstruction, chronic nasal obstruction is considered to be a result from delayed reaction mainly comprising inflammatory reaction.

Nasal obstruction symptom in allergic rhinitis is still intractable symptom, and antihistamine is hardly effective for that symptom. Although steroidal nasal drops are effective for it, but there is such a defect that the drugs hardly reach to the region, nasal mucosa due to rhinocleisis as the drugs are locally applied. Further from the viewpoint of side effects oral steroids are a little used. Cys-LT₁ receptor antagonist (anti-leukotriene) and TXA₂ receptor antagonist (anti-thromboxane) are known to be effective for nasal obstruction, but they control only single mediator, and their effectiveness is limited.
[Patent document 1] WO 02/02542

### DISCLOSURE OF THE INVENTION

The problem of the present invention is to provide a therapeutic agent useful for nasal obstruction · nasal mucosa congestive symptom in pollen allergy, eosinophile rhinitis, sinusitis, etc.

In more detail, the problem of the present invention is to provide a therapeutic agent for (1) pollen allergy, eosinophile rhinitis, acute rhinitis, chronic rhinitis, hypertrophy rhinitis, atophic rhinitis, rhinitis sicca anterior, vasomotor rhinitis or necrotizing rhinitis, etc., (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis, necrotizing rhinitis, etc., (3) sinusitis such as chronic sinusitis, intractable sinusitis, eosinophile sinusitis, nasal polyposis formation, etc., or (4) immediate nasal obstruction symptom or delayed persistent nasal obstruction associated with sinusitis. Furthermore, the problem of the present invention is to provide a therapeutic agent for asthma having higher safety and lower tonicity.

The present inventors earnestly studied for solving the above problems to find the method solving the problem by the following meanings.

Namely, it was found that the above problems were solved by using N-{2-[2-[(3-fluorophenyl)imino]-4-(4-morpholin-4-ylphenyl)-1,3-thiazol-3(2H)-yl]ethyl}-N'-methylurea represented by the formula (1) or its pharmaceutically acceptable salt as an active ingredient and then the present invention was completed.

The present invention relates to inventions illustrated below.
(1) A method for preventing and/or treating diseases related to immune comprising administering N-{2-[2-[(3-fluorophenyl)imino]-4-(4-morpholin-4-ylphenyl)-1,3-thiazol-3(2H)-yl]ethyl}-N'-methylurea represented by the following formula (1) or its pharmaceutically acceptable salt to a patient in its effective amount. 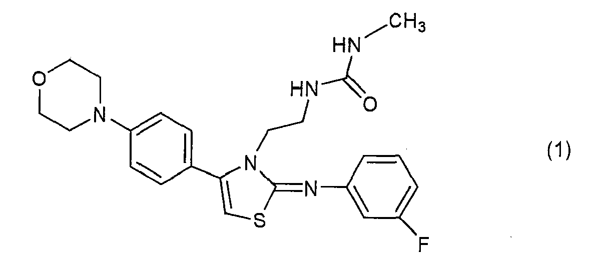
(2) A method for preventing and/or treating asthma symptom comprising administering N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt to a patient in its effective amount.
(3) A method for preventing /or treating (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis, comprising administering an effective amount of N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt to a patient.
(4) A prophylactic and/or therapeutic agent for asthma containing N-{2-[2-[(3-fluorophenyl)iminol-4-(4-morpholin-4-ylphenyl)-1,3-thiazol-3(2H)-yl]ethyl}-N'-methylurea or its pharmaceutically acceptable salt as an active ingredient.
(5) A prophylactic and/or therapeutic agent for (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis, containing, as an active ingredient, N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt.
(6) A prophylactic and/or therapeutic agent for (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis containing N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt in combination of an antihistamine or a steroid as a coadministered drug.
(7) The prophylactic and/or therapeutic agent according to above (6) wherein the coadministered drug is an antihistamine.
(8) The prophylactic and/or therapeutic agent according to above (6) wherein the coadministered drug is a steroid.
(9) Use of N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt in the manufacture of a medicament for preventing or treating asthma.
(10) Use of N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt in the manufacture of a medicament for preventing or treating (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis.

### EFFECT OF THE INVENTION

By using N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt related to the present invention, it became possible to prevent and/or treat (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis. Furthermore, by using N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt related to the present invention, it became possible to safely prevent and/or treat asthma.

### BRIEF DESCRIPTION OF DRAWINGS

Figure 1 shows effect of the drug on production and release of inflammatory mediator from mast cells derived from murine marrow.
Figure 2 shows inflammatory mediator from mast cells derived from murine marrow.
Figure 3 shows effect of the drug on production and release of inflammatory mediator from mast cells derived from guinea pig eosinophiles.
Figure 4 shows effect of the drug on variation of nasal airway resistance in guinea pig rhinitis model.
Figure 5 shows effect of the drug on variation of immediate, delayed nasal airway resistance in guinea pig rhinitis model.
Figure 6 shows effect of the drug on production and release of inflammatory mediator in nasal cavity of guinea pig rhinitis model.

### BEST MODE FOR CARRYING OUT THE INVENTION

As pharmaceutically acceptable salts of N-{2-[2-[(3-fluorophenyl)imino]-4-(4-rnorpholin-4-ylphenyl)-1,3-thiazol-3(2H)-yl]ethyl}-N'-methylurea (1) related to the present invention, there are illustrated an acid additional salt and a basic additional salt. As the acid additional salt, there are illustrated for example an inorganic salt such as hydrochloride, hydrobromide, sulfate, etc., and an organic acid salt such as citrate, oxalate, malate, oxalate, fumarate, maleate, etc. As a basic additional salt, there are illustrated an inorganic basic salt such as sodium salt, calcium salt, etc., and an organic basic salt such as meglumine salt, trishydroxymethylaminomethane salt, etc.

The compound included in the present invention may have an asymmetric carbon or a substituent having an asymmetric carbon and therefore, optical isomers can be existed. The present invention includes a mixture of each isomer or an isolated isomer. N-{2-[2-[(3-Fluorophenyl)imino]-4-(4-morpholin-4-ylphenyl)-1,3-thiazol-3(2H)-yl]ethyl}-N'-methylurea or its pharmaceutically acceptable salt related to the present invention includes also a solvate such as its hydrate.

N-{2-[2-[(3-Fluorophenyl)imino]-4-(4-morpholin-4-ylphenyl)-1,3-thiazol-3(2H)-yl]ethyl}-N'-methylurea or its pharmaceutically acceptable salt related to the present invention is useful for a medicament, and a prophylactic and/or therapeutic agent for the diseases related to immune, namely (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis, as said compound prohibits eosinophile migration·infiltration, production and/or release of inflammatory mediator from eosinophiles, T cell migration·infiltration, production and/ or release of inflammatory mediator from mast cells, production and/or release of Th2 type cytokines (IL-4, IL-5, IL-13), production of cysteinyl·lekotrienes (= leukotriene C₄/D_{4/}E₄, abbreviated as Cys-LT hereinafter), production and/or release of Tumor Necrosis Factor-α (abbreviated as TNF-α hereinafter), and production of IgE antigen. Further due to prohibiting production or release of Th2 type cytokines, said compound is also effective for diseases due to enhanced Th2 type cytokine production. As N-{2-[2-[(3-fluorophenyl)imino]-4-(4-morpholin-4-ylphenyl)-1,3-thiazol-3(2H)-yl]ethyl}-N'-methylurea related to the present invention has a mechanism different from known drugs, in case of administration for treating the above diseases, it may be coadministered with a prohibiting or therapeutic agent which is used from of old, for example, an anti-allergic agent (a chemical mediator release inhibitor, an antihistamine, an anti-leukotriene, an anti-thromboxane, or a Th2 cytokine inhibitor), a steroid (an inhaled steroid, a nasal steroid, an oral steroid, etc.), a bronchial or mycretic dilator (a βstimulant, a symphasomimetic, a parasympatholytic (an anticholinergic), etc.), a drug for vaccine therapy, a Chinese medicine, a coadministered drug thereof (a coadministered drug of an inhaled β stimulant, an inhaled steroid, etc.). Especially in case of coadministration with a steroid or a combination drug with a streroid, the therapeutic effect of the steroid or the combination drug with a streroid increases, and it is possible to decrease the amount of the steroid or the combination drug with a streroid or eliminate the steroid or the combination drug with a streroid.

The anticholinergics include for example, ipratoropium bromide, flutorpium bromide, oxitropium bromide, etc. The chemical mediator release inhibitors include for example, cromoglycic acid, sodium cromoglycate, tranilast, etc. The antihistamines include diphenhydramine, chlorpheniramine, cetirizine, loratadine, diphenylpyraline, olopatadine, bepotastine, ketotifen, terfenadine, mequitazine, azelastine, epinastine, oxagrel, fexofenadine, ebastine, oxatomide, etc. The antileukotrienes include for example, pranlukast, montelucast, etc. The Th2 cytokine inhibitors include for example, suplatast, etc.

The inhaled β stimulants include for example, salmeterol, procaterol, mabuterol, trimetoquinol, tulobuterol, theophilline, salbutamol, etc., preferably salmeterol. The steroids include for example, fluticasone, beclomethasone, prednisolone, dexamethasone, bethamethasone, etc., preferably fluticasone. The coadministered drugs of an inhaled βstimulant and an inhaled steroid include for example, a coadministered drug of salmeterol and fluticazone.

N-{2-[2-[(3-Fluorophenyl)imino]-4-(4-morpholin-4-ylphenyl)-1,3-thiazol-3(2H)-yl]ethyl}-N'-methylurea, its pharmaceutically acceptable salt and a medicament containing it which are related to the present invention are orally or parenterally administered. In case of oral administration, it is administered in a usual form. In parenteral administration, it is administered in a form of local administration (inhalations, nasal drops, external preparations), injections, percutaneous administration, nasal administration, etc. Capsules, tablets, pills, powders, caches, suppositories, solutions, etc., are illustrated as a preparation for oral administration or rectal administration. As injections, for example, steriled solution or suspension, etc. are illustrated. Creams, ointments, lotions, percutaneous agents (patches, etc.) and so on are illustrated as an agent for local administration.

The above formulations are prepared starting from the active ingredient and pharmaceutically acceptable nontoxic excipients, etc. in the usual manner. The excipients include for example, carriers, binders, perfumes, buffers, thickeners, coloring agents, stabilizers, emulsifying agent, dispersants, suspending agents, preservatives, etc. As the carriers, for example, magnesium carbonate, magnesium stearate, talc, sugar, lactose, pectin, dextrin, starch, gelatin, tragacanth, methylcellulose, sodium carboxymethylcellulose, wax with lower melting point, cacao butter, etc., are illustrated.

Capsules are prepared by putting N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt and pharmaceutically acceptable carriers into capsules, or by mixing it with pharmaceutically acceptable diluents and putting the mixture into capsules, or putting it without any carrier into capsules. Caches are also prepared with the same method.

Powders are prepared by mixing the active ingerdient and pharmaceutically acceptable bases. The bases include talc, lactose, glucose, etc. Drops are prepared by mixing the active ingredient and aqueous or nonaqueous bases and one or more pharmaceutically acceptable dispersing agents, suspending agents, solubilizer, etc.

As injectable solutions, there are illustrated solutions, suspensions, emulsions, etc., for example, aqueous solution, aqueous propylene glycol, etc. The solutions may contain water. The solution can be also prepared in a form of polyethylene glycol and/or propylene glycol solution. The solution suitable for oral administration can be prepared by adding N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt to water and if necessary, adding coloring agents, perfumes, stabilizers, sweetening agents, solubilizers, thickeners, etc., thereto. Further the solution suitable for oral administration can be prepared by adding N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt and dispersing agents to water to make viscous. The thickeners, for example, include pharmaceutically acceptable natural or synthesized gums, resins, methylcellulose, sodium carboxymethylcellulose or known suspending agents, etc.

Topical preparations include the above solutions, creams, aerosols, sprays, powders, lotions, ointments, etc. The above topical preparations are prepared by mixing N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt with a usually used diluent and carrier. The cream and ointment are prepared for example, by adding a thickener and/or gelling agent to an aqueous or oily base. The bases include for example, water, liquid paraffin, vegetable oil, etc. The thickeners include for example, soft paraffin, aluminum stearate, cetostearyl alcohol, propylene glycol, lanolin, hydrogenated lanolin, bees wax, etc. The lotions can be prepared by adding to an aqueous or oily base, at least one pharmaceutically acceptable stabilizer, suspension, emulsion, dispersion, thickener, coloring agent, perfumes, etc. The topical preparation may, if necessary, contain preservatives such as methyl hydroxybenzoate, propyl hydroxybenzoate, chlorocresol, benzalkonium chloride, or bacterial growth inhibitors. N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt can be administered via lung, inhalantly or nasally in a form of splay solutions, powders, dry powders, or drops.

The dosage and adminisration interval of compound (1) or its pharmaceutically acceptable salt vary depending on symptoms, age, body weight, administration forms, etc. In case of oral administration, the compound can be administered usually in a range of about 2 to 500mg/adult/day, preferably about 5 to 200mg, especially preferably about 10 to 100mg, in a single dose or divided doses. In case of injection, the compound can be administered in a range of about 0.1 to 300mg, preferably about 1 to 200mg, in a single dose, in divided doses or continuously (by dripping). In case of pulmonary, inhalant or nasal administration, the compound can be administered in a range of about 0.1 to 300mg, preferably about 1 to 200mg, in a single dose or divided doses. In case of external application (ointments, creams, etc.), the compound can be applied in a range of about 0.1 to 300mg, preferably about 1 to 200mg in a single dose or divided doses. In case of patches, the compound can be applied in a range of about 0.1 to 300mg, preferably about 1 to 200mg in a single dose or divided doses.

The present invention is illustrated by the following examples, but is not limited by them.

### (Test sample)

Disodium cromoglycate (DSCG, Intal inhaler, Fujisawa Pharmaceutical), dexamethasone (dex., Nakarai Tesc), AA-861 (Wako Pure Chemical) and indomethacin (Wako Pure Chemical) were commercially purchased.

### Example 1

### N-{2-[2-[(3-Fluorophenyl)imino]-4-morpholin-4-ylphenyl)-1,3-thiazol-3(2H)-yl]ethyl}-N'-methylurea (sometimes abbreviated as SMP-028)

Thi compound was prepared by the method described in WO 00/ 18399 (Patent document 1).
(1) To acetonitrile (20ml) containing t-butyl 2-(aminoethyl)carbamate (1.02g) was dropped 3-fluorophenylisothiocyanate (752mg) and the mixture was heated at 75°C for 1 hour. The mixture was concentrated in vacuo and crystallized from n-hexane to give t-butyl 2-{[(3-fluoroalinino)carbothioyl]amino}ethylcarbamate (1.81g).
   ¹H-NMR (CDCl₃): δ1.35 (9H, s), 3.35 (2H, m), 3.74 (2H, m), 4.89 (1H, bs), 6.99 (3H, m), 7.37 (1H, m), 7.81 (1H, bs)
(2) A mixture of t-butyl 2-{[(3-fluoroalinino)carbothioyl]amino}ethylcarbamate (1.81g) prepared in above (1), α-bromo-4'-morpholinoacetophenone (1.56g) and ethanol (20ml) was stirred at 45°C under nitrogen atmosphere. One hour later, resulted crystals were filtered to give tert-butyl {2-[2-[(3-fluorophenyl)imino]-4-(4-mnorpholinophenyl)thiazol-3(2H)-yl]ethyl}carbamate (1.76g). A mixture of thus obtained compound (1.76g), methanol (5ml) and 4N-HCl dioxane (50ml) was stirred at room temperature. Three hours later, the reaction mixture was concentrated in vacuo, and the insoluble materials were filtered off to give N-[3-(2-aminoethyl)-4-(4-morpholinophenyl)thiazol-2(3H)-idene]-3-fluoroaniline (1.57g). To a mixture of thus obtained compound (1.57g), aqueous 2N-sodium hydroxide solution (10ml) and tetrahydrofuran (20ml) was added phenyl N-methylcarbamate (907mg), and the mixture was stirred at 55°C. Three hours later to the reaction mixture was added water, and the mixture was extracted with ethyl acetate. An organic layer was washed with brine and dried over sodium sulfate. After removal of the solvent in vacuo, the residue was crystallized from methanol to give the titled compound (810mg) as needles.
   mp:190-191°C
   IR (KBr, cm⁻¹): 3328, 2949, 2852, 1618, 1595, 1577.
   ¹H-NMR (CDCl₃): δ2.69 (3H, d, J=4.8), 3.26 (4H, t, J=4.8), 3.45 (2H, m), 3.89-3.93 (6H, m), 5.10-5.60(2H, m), 5.80 (1H, s), 6.80-6.93 (3H, m), 6.97 (2H, d, J=8.8), 7.28-54 (3H, m).

### Example 2

### Effect of the drug on release of inflammatory mediator from murine mast cells

### (1) Study on release of inflammatory mediator from murine mast cells

According to almost the same method as the method by Razin et al. (J. Immunol. 132, 1479-1486 (1984)), murine bone marrow cells were differentiated into mast cells. Namely, bone marrow cells from femur of a female BALB/c mouse (purchased from Charles River Japan) were collected, and cultured in RPMI 1640 culture medium, containing 10% immobilized FBS prepared by Invitrogen, and 50%WEHI-3 cell culture supernatant. After culture for more than three weeks and confirming differentiation into mast cells under microscope, the cells were subjected to the following experiments.

### (2) Experiment of histamine release

Mast cells were suspended in the concentration of 2×10⁶ cell/ml, and thereto was added 0.1µg/ml anti-mouse DNP-Age antibody (SPE7, by Sigma-Aldrich). The cell suspension was incubated overnight in a CO₂ incubator (5%CO₂, 37°C), and passively sensitized. By washing with Tyrone solution (by Sigma-Aldrich) more than twice, the antibody which did not bond to the cells was removed, and the mast cells were dispersed in Tyrone solution with the concentration of 1.25×10⁶ cell/ml. The cell suspension (80µl) were added to a 96-well culture plate (by Asahi-techno-glass Co. Ltd.), and then a test sample in DMSO solution or 2.5% DMSO solution only (10µl) was added. After the plate was reinsulated in a CO₂ incubator for 10 minutes, to stimulate, 10µl of 1µg/ml of antibody (DNP-HSA, by Sigma-Aldrich, final concentration of antigen 100ng/ml) was added to each well. And then the plate was incubated in a CO₂ incubator for 20 minutes. At this time Tyrone solution was added to a Blank group. After terminating the reaction by putting the plate on ice, histamine concentration of the supernatant was measured. The measurement was conducted by using Histamine enzyme immunoassay kit (by SPI bio).

### (3) Experiment of Cyst-LT release

This experiment was conducted in the almost same method as the experiment of histamine release except that the final concentration of antigen (DNP-HSA) was 10ng/ml. The concentration of certainly·leporine in supernatant was measured by using Cysteinyl-Leukotriene EIA Kit (by Cayman Chemical).

### (4) Experiment of cytokine (TNF-α, IL-13) release

This experiment was conducted in almost the same method as the experiment on histamine release except that the final concentration of antigen (DNP-HSA) was 3ng/ml, and reaction hours after addition of the antigen were 4 hours. The concentrations of TNF-α, and IL-13 in the supernatant were measured by using Enzyme-linked immunoassay kit (Quantikine (reg. trademark) Mouse TNF-α/TNFSF1A and IL-13 Immunoassay, by R&D systems).

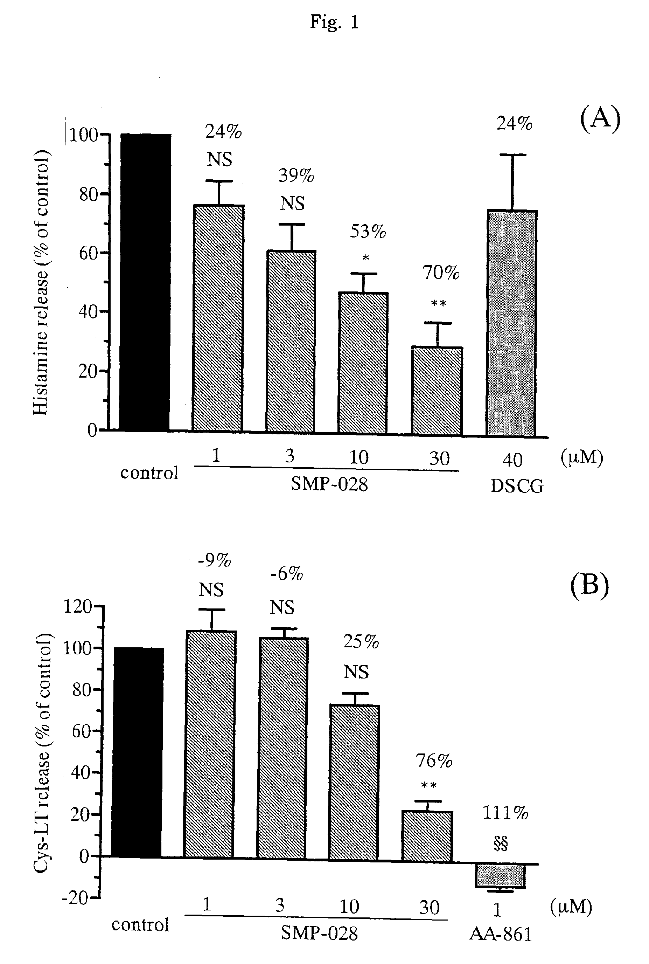

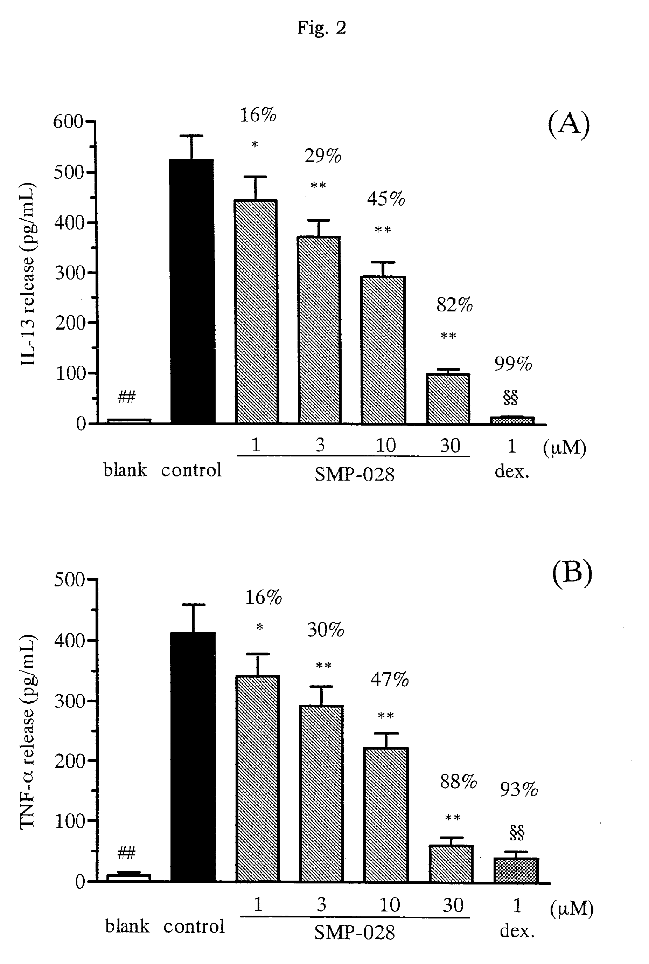

The results were shown in Figures 1 and 2.

Fig. 1 shows the effect of the drug on release and production of inflammatory mediator from mast cells derived from murine morrow. It shows the effect of SMP-028 on release of / cysteinyl·leukotriene (Cys-LT)(A) or histamine (B) from mast cells derived from murine bone morrow. The results were shown with mean ± standard error in which the tests were 5 times conducted in duplicate. Percentage shows inhibition rate. **p<0.005, *p<0.025: Comparison with control group (Williams test using raw data, one-sided test). §§p<0.01: Comparison with control group (Welch test using raw data, two-sided test)). NS: No significance against control group (Williams test using raw data, one-sided test).

Fig. 2 shows inflammatory mediator from mast cells derived from murine morrow. It shows the effect of SMP-028 on release and/or production of IL-13 (A) or TNF-α (B) from mast cells derived from murine bone morrow. The results were shown with mean ± standard error in which the tests were 6 times conducted in duplicate. Percentage shows inhibition rate. ##p<0.01, §§p <0.01: Comparison with control group (two-way layout analysis of variance, two- sided test). *p<0.05, **p<0.01: Comparison with control group (two-way layout Dunnett's test, two-sided test).

### (5) Evaluation

As shown clearly in Fig. 1 and Fig. 2, SMP-028 inhibited the release of histamine, Cys-LT, TNF-α, and IL-13 derived from mast cells. Namely it was shown that SMP-028 inhibited the release of inflammatory mediator from mast cells present in nasal cavity and nasal sinus.

### Example 3

### Effect of the drug on release of inflammatory mediator from guinea pig eosinophiles

In accordance with almost the same as the method of Sugasawa et al. (Agents and Actions 37, 233-237 (1992)), eosinophiles were separated and purified from abdominal of a male Hartley guinea pig (from SLC) and the cell concentration was adjusted to 1.25×10⁷ cell/ml with modified hanks solution. The cell suspension (80µl) were added to a 96-well culture plate (by Asahi-techno-glass Co. Ltd.), and then a test sample in DMSO solution or 2.5% DMSO solution only (10µl) was added. After the plate was preincubated in a CO₂ gas incubator for 10 minutes (5% CO₂, at 37°C), thereto was added Platelet Activating Factor (PAF C-16, by Cayman Chemical) 10µl (final concentration of PAF C-16: 10-7M), and the mixture was further incubated in a CO₂ gas incubator for 10 minutes. The concentrations of thromboxane B2 and prostaglandin in the supernatant were measured by using immunoassay kit (Thromboxane B2 EIA kit, Prostaglandin D2-MOX EIA kit, by Cayman Chemical).

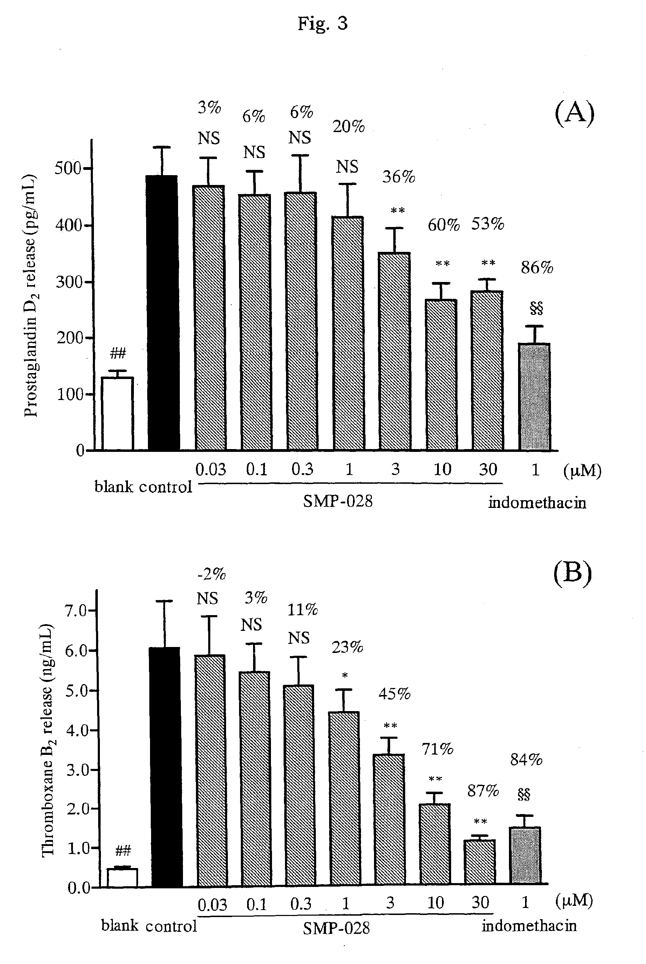

The result was shown in Fig. 3.

Fig. 3 shows the effect of the drug on release and production of inflammatory mediator from guinea pig eosinophiles. It shows the effect of SMP-028 and indomethasine on the release and production of prostaglandin D2 or thromboxatine B2 from PAF induced guinea pig eosinophiles. The results were shown with mean ± standard error in which the tests were 6 times conducted in duplicate. Percentage shows inhibition rate. ##p<0.001, §§p<0.01: Comparison with control group (two-way layout analysis of variance, two-sided test). *p<0.05, **p<0.01: Comparison with control group (two-way layout Dunnett's test, two-sided test). NS: No significance against control group (two-way layout Dunnett's test).

### Evaluation

As shown clearly in Fig. 3, SMP-028 concentration-dependently inhibited the release of prostaglandin D2 or thromboxatine B2 derived from eosinophiles. Namely it was shown that SMP-028 prohibited the release of inflammatory mediator from eosinophiles infiltrated into nasal cavity and nasal sinus.

### Example 4

### Effect of the drug on nasal cavity resistant change on guinea pig rhinitis model

### (1) Evaluation on nasal cavity resistant change on guinea pig rhinitis model

Referring to the report (74th Japanese Pharmacological Society (2001), Abstract number P-258, Establishment delayed rhinitis model by a guinea pig and effect of polysulfonic acidified hyaluronic acid, Masaki Matsudo, Junichi Fuchigami et al.), the following evaluation was conducted.

### (2) Sensitization

A saline solution (1ml) containing OVA (50mg) and aluminum hydroxide gel (1mg) (by PIERCE) was percuteneously administered to a male Hartley guinea pig (from Kyudo Co. Ltd.) on back collum to make active sensitization.

### (3) The drug administration

SMP-028 was orally administered for 15 days starting from sensitization once a day (5ml/body weight (kg)). The drug was orally administered before 1 hour on the sensitized day or on the antibody-challenged day. To saline solution challenged group (saline group) and control group was orally administered 0.5%MC as well. To dexamethasone group (dex. group) was orally administered the drug before 16 hours and 2 hours of antibody challenge.

### (4) Antibody nasal challenge

Fourteen days after sensitization, 5% OVA-saline solution (each 20µl) was nasally administered to both sides of nasal cavity by a micropipette to make antibody nasal challenge. To saline solution nasal group (saline group) was conducted the density sensitization as well, and instead of antibody nasal challenge, a saline solution was nasally administered (solvent nasal challenge).

### (5) Measurement of nasal cavity resistance

By using a general respiratory function analysis system (PULMOS-I, by M.I.P.S), nasal cavity resistance of guinea big under palinesthesia was measured in accordance with the method of Double flow plestymograph. Because antibody challenge region was restricted to nose part, sRaw which was analyzed by computer and outputted (specific airway resistance) was served as an index of nasal cavity resistance. Before antibody nasal challenge, 10 minutes, 2, 3, 4, 5, 6 and 7 hours after the challenge, each sRaw per animal was once measured, and average of 100 breathes was made sRaw at each measured time. From the sRaw value by using analysis soft on a general respiratory function analysis system (WinPUL 13 ver.1.21) and using the calculation formula (sRaw increased rate (%) during each measuring times = 100 × (sRaw-sRaw before challenge during each measuring times) / sRaw before challenge), sRaw change rate was calculated. The evaluation of the compound was conducted from sRaw changing rate at each measured time, sRaw increased rate 10 minutes later after challenge (immediate reaction, IAR, Immediate Nasal Airway Response) and Area Under the response Curve (AUC₃-₇ₕᵣ; delayed reaction, LAR, Late Nasal Airway Response) calculated from sRaw increased rate after 3-7 hours. AUC₃₋₇ₕᵣ was calculated by trapezoid (echelon) method. Furthermore, from IAR and LAR in medium control group and each test group, inhibiting rate (%) on each test group against medium control group was calculated.

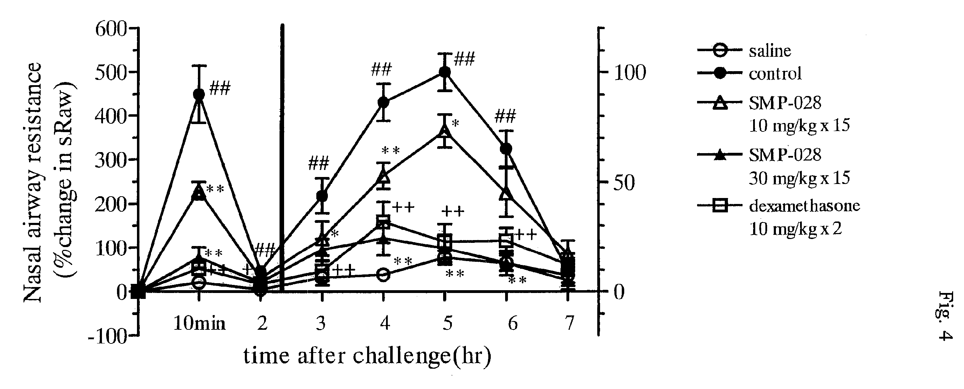

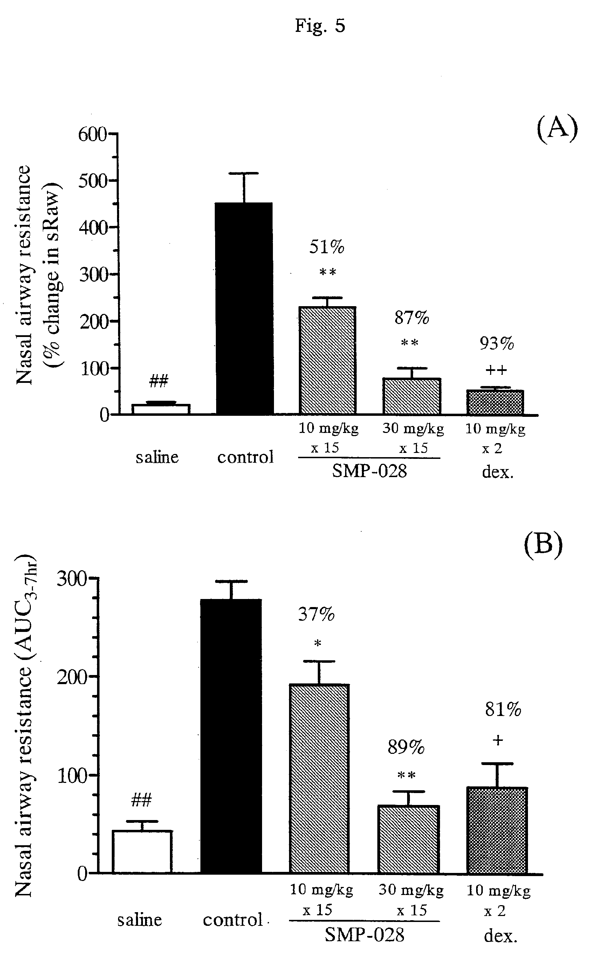

The results were shown in Fig. 4 and Fig. 5.

Fig. 4 shows the effect of the drug on nasal cavity resistance change of guinea pig rhinitis model. After antigen induction by nasal dropping on the active sensitized guinea pig, action of SMP-028 and dexamethasone to variation with time of nasal cavity resistance (sRaw) is shown in it. The result is shown with average ± standard error of 8 animals. Percentage shows inhibition rate. ##p<0.01: Comparison with control group (Student t test or Aspin-Welch test, two-sided test). *p<0.05, **p<0.01: Comparison with control group (Dannett's test, two-sided test). +p<0.05, ++p<0.01: Comparison with control group (Student t test, two-sided test)

Fig. 5 shows the effect of the drug on immediate and delayed nasal cavity resistance change of guinea pig rhinitis model. Against antigen induction immediate nasal cavity resistance reaction (IAR) and delayed nasal cavity resistance reaction (LAR) on active sensitized guinea pigs, action of SMP-028 and dexamethasone to variation with time of nasal cavity resistance (sRaw) is shown in it. The result is shown with average ± standard error of 8 animals. Percentage shows inhibition rate. ##p<0.01: Comparison with control group (Student t test, two-sided test). *p<0.05, **p<0.01: Comparison with control group (Dannett's test, two-sided test). +p<0.05: Comparison with control group (Student t test, two-sided test).

### (6) Evaluation

As shown clearly in Fig. 4 and Fig. 5, in the tests wherein a positive control, dexamethasone showed efficacy, SMP-028 dose-dependently inhibited immediate and delayed nasal congestion reaction, and it showed efficacy comparative to dexamethasone at 30mg/kg.

### (7) Nasal cavity lavage fluid

Eight hours after antigen nasal challenge, by intraperitoneally administering pentobarbital sodium (Nembutal (reg. trade name), by Dainippon Sumitomo Pharma) 30mg/body weight (kg) to anesthetize, the animal was fixed at the back. Opening bronchial, a cannula was inserted into a lung site to continue breath, and fixed by knotting. After stopping the channel of oral cavity and nasal cavity, from a part of opened bronchia to a side of nasal cavity a cannula was inserted, and knotted, and to the cannula was connected syringe pump. A saline solution was refluxed in the nasal cavity at flow speed (1ml/minute) for 10 minutes, and the refluxed solution was recovered to get nasal cavity lavage fluid, NCLF). NCLF was centrifuged (1710.5 × g (3000rpm), 4°C, 10minutes), and the supernatant was freezed at -80°C, and subjected to experiment of Example 5.

### Example 5

### Effect of the drug on production of inflammatory mediator in nasal cavity of guinea pig rhinitis model

### (1) Measurement of inflammatory mediator in nasal cavity

The supernatant of NCLF obtained in Example 4 was freezed at -80°C and preserved. After thawing the sample, the concentration of LTE₄ was measured by immuno assay kit (Leukotriene E₄ EIA kit, by Cayman Chemical). The concentration of TNF was measured by bioassey method using L929 cells (from Dainippon Sumitomo Pharma)(David H., et al., J. Allergy Clin. Immunol. 89, 958-967 (1992)). Using recombinant human TNF-α (by PeproTech) as a standard, the result was shown by potency of human TNF-α cytotoxicity.

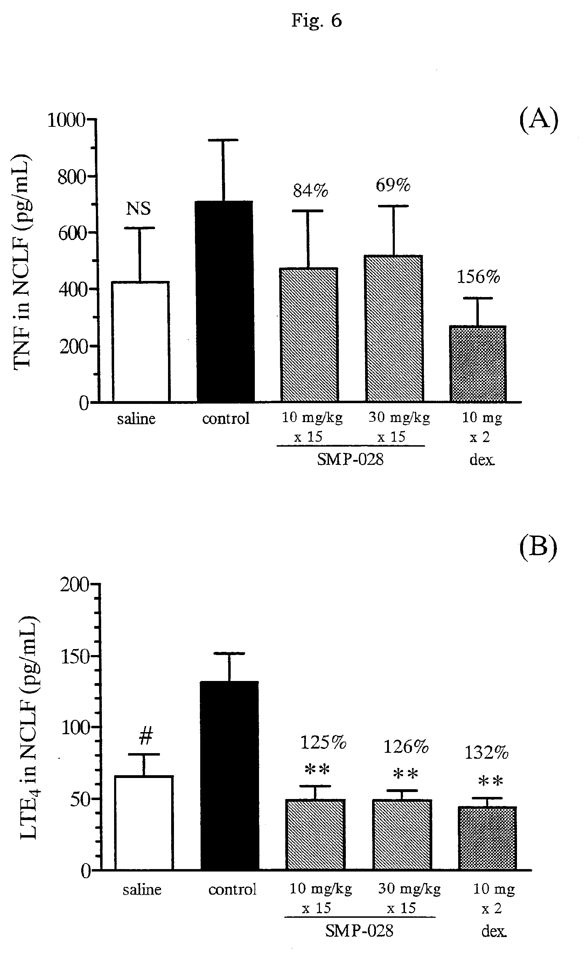

The result was shown in Fig. 6.

Fig. 6 shows the effect of the drug on production of inflammatory mediator in nasal cavity of guinea pig rhinitis model. It shows the effect of SMP-028 and dexamethasone against inflammatory mediator, (A) TNF or (B) LTE₄ in nasal cavity lavage fluid (NCLF) for 8 hours of antigen induction by nasal dropping to the active sensitized ginea pigs. The result is shown average ±standard error of 8 animals. Percentage shows inhibition rate. #p<0.05: Comparison with control group (student t-test, two sided test). **p<0.01: Comparison with control group (Dannett's test, two-sided test). NS: No significant against control group (Student t test, two-sided test) (2) Evaluation

As shown clearly in Fig. 6, SMP-028 showed a tendency and activity of inhibition of production of TNF and LTE₄ in nasal cavity.

### Example 6

### Evaluation of compounds on guinea pig delayed asthma model

### Evaluated compounds

### Compound 2: WO 02/02542 (Patent literature 1) Example 350

### Compound 1 (SMP-028): Example 1

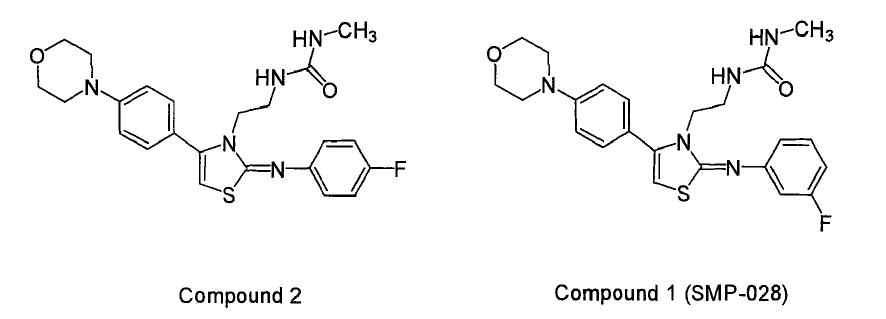

### Evaluation method

Hartley male guinea pigs were purchased from SLC, and after quarantining them for 1 week, at the week when the qualantine was finished the first sensitization was carried out by antigen exposure. By using ultrasonic nebulizer (OMRON NE-U12, condition: Vapor amount: maximum, Air: small) 2% (w/v) ovalbuminn (OVA, by Sigma-Aldrich) in saline solution was vaporized, and by disposing for 10 minutes, they were first sensitized. At sensitizing, the guinea pigs were fixed on a body holder cartridge for them (by M.I.P.S.), and they were set on disposal chamber holder for 8 guinea pigs (by M.I.P.S.). Additional sensitization was carried out 7 days after the first sensitization as well. Fourteen to sixteen days after the first sensitization, asthma reaction was induced by antigen inhalation. Before 1 hour of the induction, an antihistamine, pyrilamine maleate (in a saline solution, 10mg/2mL/kg) was intraperitoneally administered, followed by oral administration of the test sample. The test sample was prepared with aqueous 0.5% methylcellulose (MC), and its 5mL/kg was administered. To disease induction group was administered 0.5%MC as well. A 2%OVA solution (OVA by Seikagaku Corp.) was vaporized by super ultrasonic wave nebulizer, and by inhalating it for 5 minutes, asthma reaction was induced. Breath function was measured by a general breath function measurement system (by M.I.P.S.) using Double flow plestymograph method (Penny A. Hutson et al. Am Rev Respir Dis 1988 137, 548-557). The breath function was measured before reaction induction (pre-value), 5 minutes, 1, 2, 3, 4, 5, 6, 7, and 8 hours after completion of reaction induction. By using one of parameters, sRaw/TV (specific airway resistance/once breath) as an index of improvement of respiratory function, variation rate per individual of sRaw/TV (%sRaw/TV) against pre-value between each measured times was calculated. Plotting %sRaw/TV with time, Area Under the Curve (AUC₄₋₈ₕᵣ) from 4 hours to 8 hours after reaction induction was calculated. By using the AUC₄₋₈ₕᵣ value, the inhibition rate of delayed asthma reaction on each individual was calculated. Based on these values, ED₅₀ (mg/kg) values indicated in Table 1 were obtained.

**Table 1**

| | ED₅₀(mg/kg) |
|---|---|
| Compound 2 | 0.69 |
| Compound 1 (SMP-028) | 0.44 |

### INDUSTRIAL APPLICABILITY

To a patient suffered from (1) pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, etc., (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) sinusitis such as chronic sinusitis, intractable sinusitis, eosinophile sinusitis or nasal polyp sis formation, etc., or (4) immediate nasal obstruction symptom or delayed persistent nasal obstruction associated with sinusitis is administered compound (1) or its pharmaceutically acceptable salt in a single dose or divided doses to be treated without side effects.

Furthermore, to a patient suffered from asthma symptom is administered the compound in a single dose or divided doses to be treated without side effects.

## Claims

1. A method for preventing or treating a disease related to immune comprising administering N-{2-[2-[(3-fluorophenyl)imino]-4-(4-morpholin-4-ylphenyl)-1,3-thiazol-3(2H)-yl]ethyl}-N'-methylurea represented by the following formula (1): 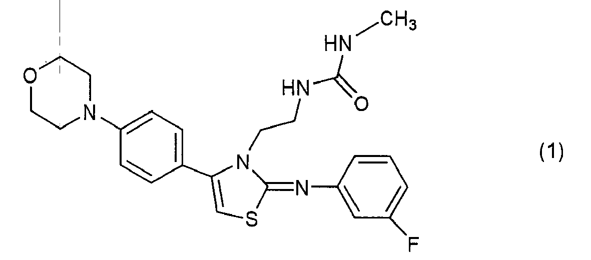 or its pharmaceutically acceptable salt to a patient in its effective amount.

2. A method for preventing or treating asthma symptom comprising administering N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt to a patient in its effective amount.

3. A method for preventing or treating (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis, comprising administering an effective amount of N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt to a patient.

4. A prophylactic or therapeutic agent for asthma containing N-{2-[2-[(3-fluorophenyl)imino]-4-(4-morpholin-4-ylphenyl)-1,3-thiazol-3(2H)-yl]ethyl}-N'-methylurea represented by the following formula (1): 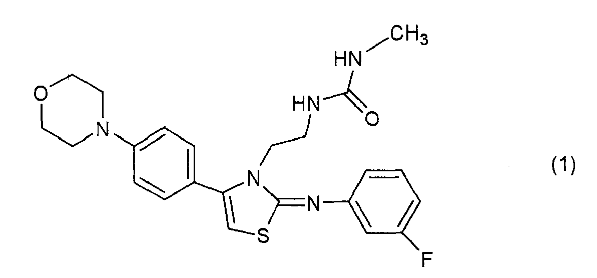 or its pharmaceutically acceptable salt as an active ingredient.

5. A prophylactic or therapeutic agent for (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis, containing, as an active ingredient, N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt.

6. A prophylactic or therapeutic agent for (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis containing N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt in combination of an antihistamine or a steroid as a coadministered drug.

7. The prophylactic or therapeutic agent according to claim 6 wherein the coadministered drug is an antihistamine.

8. The prophylactic and/or therapeutic agent according to claim 6 wherein the coadministered drug is a steroid.

9. Use of N-{2-[2-[(3-fluorophenyl)imino]-4-(4-morpholin-4-ylphenyl)-1,3-thiazol-3(2H)-yl]ethyl}-N'-methylurea represented by the following formula (1): 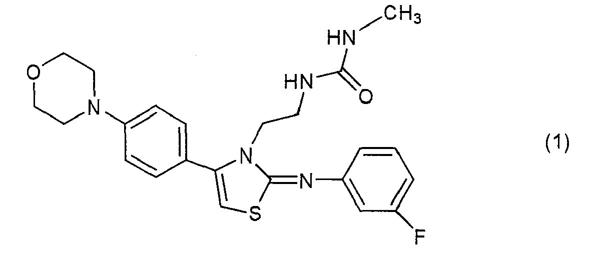 or its pharmaceutically acceptable salt in the manufacture of a medicament for preventing or treating asthma.

10. Use of N-{2-[2-[(3-fluorophenyl)imino]-4-(4-morpholin-4-ylphenyl)-1,3-thiazol-3(2H)-yl]ethyl}-N'-methylurea or its pharmaceutically acceptable salt in the manufacture of a medicament for preventing or treating (1) pollen allergy, eosinophile rhinitis, acute rhinitis, chronic rhinitis, hypertrophic rhinitis, atophic rhinitis, rhinitis sicca anterior, vasomotor rhinitis, or necrotizing rhinitis, (2) immediate nasal obstruction symptom or delayed persistent nasal obstruction associated with pollen allergy, eosinophile rhinitis, acute rhinitis, chronic rhinitis, hypertrophic rhinitis, atophic rhinitis, rhinitis sicca anterior, vasomotor rhinitis or necrotizing rhinitis, (3) chronic sinusitis, intractable sinusitis, eosinophile sinusitis, or nasal polyposis formation, or (4) immediate nasal obstruction symptom or delayed persistent nasal obstruction associated with sinusitis.
